(19) Europäisches Patentamt / European Patent Office / Office européen des brevets

(11) **EP 1 845 797 B1**

(12) **EUROPEAN PATENT SPECIFICATION**

(45) Date of publication and mention
of the grant of the patent:
**18.03.2009 Bulletin 2009/12**

(51) Int Cl.:
*A23C 9/13* (2006.01)          *A23L 1/30* (2006.01)
*A61K 31/21* (2006.01)        *A23L 1/305* (2006.01)
*A61K 38/01* (2006.01)

(21) Application number: **06706378.4**

(22) Date of filing: **24.01.2006**

(86) International application number:
**PCT/EP2006/000586**

(87) International publication number:
**WO 2006/084573 (17.08.2006 Gazette 2006/33)**

(54) **FOOD PRODUCTS COMPRISING HYDROLYSED MILK SOLIDS WITH IMPROVED TASTE**

LEBENSMITTELPRODUKTE MIT HYDROLYSIERTEN MILCHFESTSTOFFEN MIT
VERBESSERTEM GESCHMACK

PRODUITS ALIMENTAIRES A GOUT AMELIORE COMPRENANT DE LA MATIERE SECHE
OBTENUE APRES HYDROLYSE DU LAIT

(84) Designated Contracting States:
**AT BE BG CH CY CZ DE DK EE ES FI FR GB GR
HU IE IS IT LI LT LU LV MC NL PL PT RO SE SI
SK TR**

(30) Priority:  **09.02.2005   EP 05075323**

(43) Date of publication of application:
**24.10.2007   Bulletin 2007/43**

(73) Proprietors:
• **Unilever N.V.
3013 AL Rotterdam (NL)**
Designated Contracting States:
**AT BE BG CH CZ DE DK EE ES FI FR GR HU IS IT
LI LT LU LV MC NL PL PT RO SE SI SK TR**
• **Unilever PLC
London
EC4Y 0DY (GB)**
Designated Contracting States:
**CY GB IE**

(72) Inventors:
• **VAN BENTHUM, Wilhelmus,
Unilever R&D Vlaardingen
NL-3133 AT Vlaardingen (NL)**
• **SCHALK, Johannes,
Unilever R&D Vlaardingen
NL-3133 AT Vlaardingen (NL)**

(74) Representative: **Joppe, Hermina Laura Petronella
et al
Unilever Patent Group
Olivier van Noortlaan 120
3133 AT Vlaardingen (NL)**

(56) References cited:
**EP-A- 0 583 074          EP-A- 1 212 945
WO-A-03/055324          WO-A-20/04014141
US-B1- 6 391 370          US-B1- 6 677 327**

• **VOLPE R ET AL: "EFFECTS OF YOGHURT
ENRICHED WITH PLANT STEROLS ON SERUM
LIPIDS IN PATIENTS WITH MODERATE
HYPERCHOLESTEROLAEMIA" BRITISH
JOURNAL OF NUTRITION, CAMBRIDGE, GB, vol.
86, no. 2, August 2001 (2001-08), pages 233-239,
XP008024215**
• **SEPPO L ET AL: "The effect of a Lactobacillus
helveticus LBK-16 H fermented milk on
hypertension - a pilot study on humans"
MILCHWISSENSCHAFT, VV GMBH
VOLKSWIRTSCHAFTLICHER VERLAG.
MUNCHEN, DE, 2002, pages 124-127,
XP002243244 ISSN: 0026-3788**

## Description

### Field of the invention

The invention relates to food products comprising hydrolysed milk solids and a sterol fatty acid ester. These products are effective in reducing hypertension.

### Background to the invention

Hypertension is very common in the western society. In the year 1999, in the USA more than 25% of the people have above normal blood pressure, caused by the western lifestyle. Hypertension is considered to be one of the main causes of cardiovascular hearth disease (CVD).

Long term human studies have shown that regular intake of low amounts of hypertension lowering drugs reduces CVD with 25% (Gerstein et al. (2000), The Lancet 355, 253-259). Some of these drugs are based on hydrolysed proteins, especially hydrolysed casein.

EP-A-821968 discloses an antihypertensive agent that may be obtained by culturing a peptide and/or protein containing product such as animal milk with lactic acid bacteria, especially those of the genus *Lactobacillus*.

EP-A-1365656 discloses a fermented milk product that has a hypertension lowering effect. This document discloses that milk, fermented with specific *Lactobacillus* strains, *Lactobacillus helveticus,* may have a number of undesired properties such as an unacceptable taste. This taste may partly be due to acidic pH and to a "cheesy" off taste that is linked to fermented milk products. EP-A-1365656 discloses that the taste may be improved by using a specific strain, *Lactobacillus* delbrueckii subspecies *lactis.*

WO-A-03/055324 also discloses that taste problems are encountered with the use of protein hydrolysates in food products. A bitter taste is reported. In WO-A-03/55324 this is solved by using at least two cholesterol lowering agents in a food product. Thus a lower protein hydrolysate content can be applied without loss of the desired cholesterol lowering effect. A further measure that is suggested is the complexing of protein hydrolysates with emulsifiers or with plant sterols. The examples disclose preparation of complexes of soy protein hydrolysate, emulsifier and free plant sterol.

WO-A-04/098309 discloses hydrolysed casein products comprising tripeptides VPP (peptide Val-Pro-Pro), IPP (peptide Ile-Pro-Pro) and/or LPP (peptide Leu-Pro-Pro). It is disclosed in the examples that yoghurt drinks comprising these hydrolysed peptides have a good taste and no bitter taste was noticed. We have however found that the application of these hydrolysed milk solids in some types of food products is accompanied by an unpleasant off taste. Depending on the process by which such hydrolysed milk solids are obtained this off taste may be characterized as cardboard, medicinal, acid dairy or "cheesy" off taste.

Milk protein hydrolysates may generally be obtained via two routes. One route is enzymatic treatment. In an example of an enzymatic treatment a substrate containing casein is treated with proteinase to produce an intermediate peptide. This intermediate peptide is further treated with a peptidase. WO-A-01/034828 discloses an example of such a process. We have found that peptides that are produced by this treatment may suffer from an off taste that is characterized by a trained panel as "cardboard, medicinal and/or acid dairy". This was especially found for emulsions comprising these hydrolysed casein products.

In an alternative process casein hydrolysates are obtained by fermentation of a casein containing substrate such as milk by specific strains such as *Lactobacillus helveticus*. The products obtained with this process may suffer from a "cheesy" off taste.

The objective of the current invention is to provide a food product comprising hydrolysed milk solids , with a reduced off taste and off smell and better mouthfeel compared to the known products.

### Summary of the invention

We have surprisingly found that the use of sterol fatty acid esters in an emulsion comprising hydrolysed milk solids leads to products with reduced off taste and off smell and with a good mouthfeel.

Therefore the invention relates to a food product comprising hydrolysed milk solids , wherein the food product further comprises a sterol fatty acid ester, as defined in claim 1.

The invention further relates to the use of a composition comprising hydrolysed milk solids and/or a peptide selected from the group comprising tripeptides VPP, IPP , LPP or a combination thereof, and a sterol fatty acid ester for the preparation of a food product for the reduction of cholesterol and/or for blood pressure lowering.

In another aspect the invention relates to the use of a sterol fatty acid ester to reduce off taste, especially cardboard off taste in a food product, especially an emulsion, comprising hydrolysed milk solids and/or a peptide selected from the group consisting of tripeptides VPP, IPP , LPP or a combination thereof.

In another aspect the invention relates to use of a sterol fatty acid ester to reduce cheesy off taste in a food product, especially an oil and water emulsion, comprising hydrolysed milk solids and/or a peptide selected from the group comprising tripeptides IPP, VPP, LPP or a combination thereof.

## Detailed description of the invention

The weight percentages herein will be expressed relative to the total weight, unless otherwise indicated.

The common one letter code is ordinarily used to describe amino acids.

Hydrolysed milk solids herein are understood to include all types of hydrolysed milk proteins, like whey protein and/or casein. Hydrolysed milk solids will often include peptides such as VPP, IPP and/or LPP.

Casein is herein understood to include all types of casein, including alpha casein, beta casein, gamma casein and kappa casein. It is noted that the fragment IPP occurs in both beta- and kappa-casein. The fragments VPP and LPP both occur once in beta casein.

Tripeptides VPP, IPP and/or LPP as defined herein include VPP, IPP, LPP and mixtures of these peptides. The total amount of tripeptides VPP, IPP and/or LPP in mixtures are herein calculated by addition of amounts of the tripeptides in the mixture.

These active tripeptides VPP, IPP, and LPP, each have a different activity in blood pressure lowering. This is generally known in the art.

The hydrolysed milk solids or tripeptides VPP, IPP, LPP, in the products according to the invention may be obtained by any suitable process. It is however preferred that they are obtained by enzymatic treatment of a substrate containing casein. Examples of a suitable enzymatic treatment are disclosed in WO-A-04/098309, EP-A-1231279 (WO-A-01/34828) and in Mizuno S et al (2004) J Dairy Sci 87: p3183-3188. Suitable fermentation conditions are for example disclosed in EP-A-583074, WO-A-04/060073A1 and in Nakamura Y et al (1995)J Dairy Sci 78: p777-783.

The substrate for fermentation or enzymatic treatment may be any casein and/or casein fragment containing material suitable as the basis for human food.

Preferably the substrate is milk. Animal milk such as cow's milk, goat's milk, sheep's milk, camel milk, horse's milk, may be used as substrate. Skim milks may be used. The content of the solid in the substrate is not particularly limited, but is usually 5 to 20wt%. The substrate may be reconstituted milk, prepared by mixing water and milk ingredients, for instance (skim) milk powder. The substrate may contain additives, such as carbohydrates, etc. as long as these additives do not interfere with the enzyme treatment and /or fermentation. Preferably caseinates, such as for instance potassium-caseinate and/or calcium caseinate may be used in a substrate.

To express the overall concentration of active tripeptides containing a branched chain amino acid -Proline-Proline sequence e.g. IPP and VPP, the equivalent LTP is also used herein, which is defined as follows and expressed in mg/100g:

$$[LTP] = [VPP] + 1.7 * [IPP]$$

LPP is not included in above [LTP] formula. In addition to VPP and IPP, LPP is preferably present in VPP/LPP ratio's between 0.001 and 50, more preferred between 0.1 and 30, even more preferred between 0.1 and 5 for enzymatically hydrolysed milk solids and between 1 and 50 for fermentative hydrolysed milk solids.

Preferred products comprise at least 0.05% hydrolysed milk solids, more preferred at least 0.1% hydrolysed milk solids, even more preferred from 0.1 to 15% hydrolysed milk solids, most preferred from 0.1 to 10% hydrolysed milk solids.

In one embodiment of this invention, the products according to the invention comprise at least 0.5 mg/100g LTP, more preferred at least 3.5 mg/100g LTP, even more preferred from 3.5 to 50 mg/100g LTP, most preferred from 3.5 to 25 mg/100g LTP on total product weight. Such LTP levels may be obtained from hydrolysed milk solids in one embodiment or via an alternative process.

The products according to the invention comprise a sterol and/or stanol fatty acid ester. In this application where reference is made to sterolester, this also includes their saturated derivatives, the stanol esters, and combinations of sterol- and stanol esters.

These compositions were unexpectedly found to reduce the specific off taste that was found when the specified hydrolysed milk solids, respectively tripeptides are applied in emulsions.

The inclusion of these compounds gives rise to another advantage that may be highly desirable. It is generally known that sterolesters may be used to lower blood cholesterol levels. Reference is for example made to EP594612 in this context. The blood cholesterol lowering effect, in addition to the blood pressure lowering effect attributable to the hydrolysed milk solids and/or the tripeptides may lead to a further reduced risk on cardiovascular diseases (CVD).

Hypertension and high blood cholesterol levels are among the main risk factors for CVD. This is another beneficial aspect of the products according to the invention.

Sterols or phytosterols, also known as plant sterols or vegetable sterols can be classified in three groups, 4-desmethylsterols, 4-monomethylsterols and 4,4'-dimethylsterols. In oils they mainly exist as free sterols and sterol esters of fatty acids although sterol glucosides and acylated sterol glucosides are also present. There are three major phytosterols namely beta-sitosterol, stigmasterol and campesterol. Schematic drawings of the components meant are as given in "Influence of Processing on Sterols of Edible Vegetable Oils", S.P. Kochhar; Prog. Lipid Res. 22: pp. 161-188.

The respective 5 alpha- saturated derivatives such as sitostanol, campestanol and ergostanol and their derivatives are in this specification referred to as stanols.

Preferably the esterified sterol or stanol is selected from the group comprising fatty acid ester of β-sitosterol, β-sitostanol, campesterol, campestanol, stigmasterol, brassicasterol, brassicastanol or a mixture thereof.

The sterols or stanols are at least partly esterified with a fatty acid. Preferably the sterols or stanols are esterified with one or more $C_{2-22}$ fatty acids. For the purpose of the invention the term $C_{2-22}$ fatty acid refers to any molecule comprising a $C_{2-22}$ main chain and at least one acid group. Although not preferred within the present context the $C_{2-22}$ main chain may be partially substituted or side chains may be present. Preferably, however the $C_{2-22}$ fatty acids are linear molecules comprising one or two acid group(s) as end group(s). Most preferred are linear $C_{8-22}$ fatty acids as these occur in natural oils.

Suitable examples of any such fatty acids are acetic acid, propionic acid, butyric acid, caproic acid, caprylic acid, capric acid. Other suitable acids are for example citric acid, lactic acid, oxalic acid and maleic acid. Most preferred are myristic acid, lauric acid, palmitic acid, stearic acid, arachidic acid, behenic acid, oleic acid, cetoleic acid, erucic acid, elaidic acid, linoleic acid and linolenic acid.

When desired a mixture of fatty acids may be used for esterification of the sterols or stanols. For example, it is possible to use a naturally occurring fat or oil as a source of the fatty acid and to carry out the esterification via an interesterification reaction.

In a preferred embodiment, the fatty acid in the esterified sterol or stanol is derived from sunflower oil, rapeseed oil, safflower oil, coconut oil, or a mixture thereof.

Most preferred the fatty acid in the esterified sterol or stanol is derived from sunflower oil.

Optionally the products comprise a combination of a sterol fatty acid ester and a stanol fatty acid ester.

Optionally the products comprise unesterified "free", sterol or stanol in addition to the esterified sterol or stanol. It is preferred that the total level of free sterol or stanol is below 50wt% on total weight of the sterol/stanol and their esters.

The total amount of sterol and stanol fatty acid ester is preferably from 0.1 to 20 wt%. More preferred, the amount is from 0.5 to 20 wt%, even more preferred from 1 to 20 wt%, even more preferred 1 to 15 wt%, most preferred from 1 to 10 wt% on total product weight. We surprisingly found that the known fatty mouthfeel of sterolesters, which is e.g. referred to in WO-A-2004/014141 is present to a much lower extent than expected on the basis of the total amount of sterolesters that is present in the products according to the invention. Without wishing to be bound by any theory, this is expected to be caused by the simultaneous presence of hydrolysed milk solids and especially the smaller peptides amongst which the tripeptides defined above.

The food products may be any products such as spreads, drinks, yoghurts, bars. Examples of drinks are milk, fruit juice products, tea, coffee.

The products according to the invention are preferably emulsions of an oil phase and an aqueous phase. Examples of food compositions that are oil and water emulsions are milk, dairy spreads, crème fraiche, cream cheese, milk type drinks and yoghurt, ice cream, sauces, cooking cream, sour cream, and spreads such as margarine, low fat spreads and butter.

The amount and type of oil in these emulsions may vary widely. It was found beneficial that at least some oil is present. Therefore the level of oil in the preferred spreadable emulsions is preferably from 1 to 60 wt%, more preferred from 5 to 50 wt%, even more preferred from 10 to 40 wt% fat.

The level of oil and fats in milk type drinks and yoghurt products is preferably below 10 wt%, more preferred below 5 wt%, even more preferred below 4 wt% fat.

The type of fat may be any fat e.g. butter fat, vegetable fat or fish oil. Products comprising butter fat and/or vegetable fat are preferred.

Preferably, the food product comprises 50-200 mmol/kg $K^+$ and/or 15-60 mmol/kg $Ca^{2+}$ and/or 6-25 mmol/kg $Mg^{2+}$ more preferably, 100-150 mmol/kg $K^+$ and/or 30-50 mmol/kg $Ca^{2+}$ and/or 10-25 mmol/kg $Mg^{2+}$ and most preferably 110-135 mmol/kg $K^+$ and/or 35-45 mmol/kg $Ca^{2+}$ and/or 13-20 mmol/kg $Mg^{2+}$.

In another preferred embodiment the products comprise an ingredient selected from the group comprising B vitamines such as Vitamin B6, B11, B12, folic acid or a combination thereof.

Optionally the products comprise a tocopherol and/or tocotrienol.Preferably the tocopherol or tocotrienol is selected from the group comprising alpha-, beta-, gamma-, delta-tocotrienol and alpha-, beta-, gamma-, delta- tocopherol.

Optionally the products comprise further ingredients such as thickeners, emulsifier, sugar, protein, taste and flavour modifiers, fruit or fruit concentrate, acidifier, cultures.

Preferably the products comprise protein, more preferably in an amount of from 0.5 to 10 wt%. The most preferred protein is milk protein such as derived from fresh milk, yoghurt, quark, skimmed milk powder, whey powder, caseinate, cream. An alternative protein source is vegetable protein, preferably soy protein.

Preferred products comprise living bacteria (live cultures). In this context live cultures are cultures which were not inactivated by a heating step. Such cultures are also referred to as yoghurt cultures and/or probiotics. Suitable cultures include *Lactobacillus, Lactococcus, Streptococcus* and *Bifidobacterium* species. It was found that such cultures may also positively contribute to off taste reduction.

Most preferred the products are drinks comprising live cultures, hydrolysed casein, up to 5 wt% fat, from 1.5 to 4 wt% sterol fatty acid ester, milk protein, water, a sugar and preferably emulsifier.

Other preferred products are water-continuous products including spreads with or without live cultures comprising hydrolysed milk solids, up to 40 wt% fat, from 1.5 to 4 wt% sterol fatty acid ester, milk protein, water and sugar and preferably emulsifier.

Even more preferred those products are acidified, preferably by fermentation with a pH in the range from 3.8 to 6, preferably from 4 to 5.

In another aspect the invention relates to use of a composition as in claim 7 comprising hydrolysed milk solids and a sterol fatty acid ester for the preparation of a food product for the reduction of cholesterol levels in blood and/or blood pressure lowering.

In a further aspect the invention relates to use of a sterol fatty acid ester to reduce off taste, especially cardboard off taste in a food product as in claim 1, especially an emulsion comprising enzymatically hydrolysed milk solids, comprising a peptide selected from the group comprising tripeptides VPP, IPP, LPP or a combination thereof.

In a further aspect the invention relates to use of a sterol fatty acid ester to reduce cheesy off taste in a food product, comprising hydrolysed milk solids.

In a further aspect the invention relates to use of a sterol fatty acid ester to reduce cheesy off taste in a food product, especially an oil and water emulsion comprising a peptide selected from the group comprising tripeptides IPP, VPP , LPP or a combination thereof.

The food compositions may be prepared by any suitable process. The hydrolysed milk solids may be added as separate ingredients or may be formed in situ during the preparation of the food composition. Methods to make hydrolysed milk solids are described in the above mentioned references.

The above-mentioned preferred embodiments for food products comprising hydrolysed milk solids, likewise apply to food products comprising a peptide selected from the group comprising VPP, IPP, LPP or a combination thereof.

The invention is illustrated by the following non-limiting examples.

## Examples

General

Determination of amounts of IPP, LPP and VPP

Quantification of IPP, LPP and VPP was performed using HPLC-MRM-MS in positive ESI mode. The samples were analysed using a HP1100 (ex Agilent) HPLC system combined with a Quattro-II triple quadrupole mass spectrometer (ex Micromass UK). The samples were injected on a Varian 150x2.1 mm Inertsil ODS-3 column prepacked by GL_ Sciences. The eluent gradient was linear from 100% water containing 0.1% trifluoric acid (TFA) to 70% of acetonitrile containing 0.1% of TFA in 40 minutes with a flow rate of 0.2 ml/min at a column temperature of 60 °C. The ion source of the MS was operating in positive-electrospray mode. The product ions m/z 213.1 and m/z 183.1 were monitored in MRM of the precursor ion m/z 326.3 for IPP and LPP, m/z 213.1 and m/z 169.1 for VPP. U13C-VPP and U13C-IPP were used as internal standards with precursor ions of m/z 317.2 and m/z 332.2 respectively. The productions monitored were m/z 213.1 and m/z 174.1 for U13C-VPP and m/z 213.1 and m/z 189.1 for U13C-IPP. The cone voltage and collision energy was 20 V and 20 eV for all compounds. The collision gas used was Argon, and the collision gas pressure was 2.3 x 10-3 mbar. The quantification was performed using a separate relative internal calibration curve for both compounds.

## Example 1

Vegetable Dairy Spread with hydrolysed milk solids.

The hydrolysed milk solids were prepared by the process as described in Mizuno S et al (2004) J Dairy Sci 87: p3183-3188. The enzyme used was Sumizyme™ FP and the hydrolysis time was about 14 hours.

Production of two standard vegetable dairy spreads (VDS) with on top 3.1% hydrolysed milk solids and a VDS with on top 3.1% hydrolysed milk solids and 3.75 gram / 100 g sterolesters (3.75 wt%)

Preparation VDS-standard process on lab-scale is done in the following way. Gelatine was together with the salt swollen in water (10%sol.) for 30 minutes at room temperature. The mixture was mixed in a vessel (Stephan™), and heated to 60°C at 300 rpm. 5% of total, pre-blended fat blend was added together with the powders (SMP, Nutrilac, LBG, K-sorbate and hydrolysed milk powder). The powders were dissolved and vegetable fat with beta-carotene and sterol esters were adedd to the mixing vessel. The mixture was heated to 85°C and hold at 85°C for 14 minutes while stirring. The gelatine/salt solution was added and mixed for 1 minute while stirring. This mixture was homogenises at 300 bar, and acid was added. Further flavours were added and the mixture was finally homogenised at 200 bar. The emulsion was filled in cups (at 72°C ) and sealed. Samples were cooled to 5°C.

Ingredients:

| | VDS-1 | Comparative example (no sterolester) |
|---|---|---|
| Fat blend comprising coconut oil with a slip melting point of 31 °C, and a palm oil fraction. Iodine value of the blend is 55. | 22 % | 22 % |
| Whey Powder (Nutrilac™ QU6560) | 1.75 % | 1.75 % |
| Skim milk powder | 6.25 % | 6.25 % |
| Locust bean gum | 0.3 % | 0.3 % |
| Gelatine 250 bloom | 0.7 % | 0.7 % |
| Salt | 0.3 % | 0.3 % |
| potassium sorbate | 0.1 % | 0.1 % |
| 50% citric acid solution | 0.6 % | 0.6 % |
| Flavour | 0.1 % | 0.1 % |
| hydrolysed milk solids | 3.1 % | 3.1 % |
| Sterol ester (mostly betasitosterol esterified with sunflower oil) | 3.75% | 0% |
| water | to 100% | to 100% |

The products comprised 20mg LTP per 100g total product.

Product tasting

The products were tasted by an expert panel, and the taste of the products could be described as cheesy and cardboard. This cheesy and cardboard taste can be ascribed to the hydrolysed milk solids. The people in the taste panel compared the taste of the 2 vegetable dairy spreads VDS-1 and the comparative spread, by tasting the spread pure. According to the panel members, the VDS-1 spread showed less cheesy and cardboard off-taste than the comparative spread.

**Example 2**

Composition:

| ingredient | Comparison fcs-1 | Comparison fcs-2 | fcs-3 (according to the invention) |
|---|---|---|---|
| fat blend of 83% sunflower oil and 17% interesterified blend of fractionated palm oil and palm kernel oil. | 37.45% | 37.45% | 0% |
| fat blend of 56% sunflower oil, 12% interesterified blend of fractionated palm oil and palm kernel oil and 32% plant sterol esters | 0% | 0% | 43% |
| lecithin | 0.15% | 0.15% | 0.15% |
| Monoglyceride (hymono™ 8903) | 0.2% | 0.2% | 0.2% |
| flavour | 0.1% | 0.1% | 0.1% |
| water | 55.3% | 52.8% | 47.3% |
| sodium chloride | 0.5% | 0.5% | 0.5% |
| potassium sorbate | 0.1% | 0.1% | 0.15% |
| sweet buttermilk powder (BMP) | 0.1% | 0.1% | 0% |
| Purity™ LFS starch | 6% | 6% | 6% |
| hydrolysed milk solids | 0% | 2.5% | 2.5% |
| pH water phase | 5.1 (with citric acid mono-hydrate) | 5.1 (with citric acid mono-hydrate) | 5.1 (with citric acid mono-hydrate) |

Fat-continuous spreads (like normal light margarines) containing plant sterol esters and hydrolysed milk solids. The hydrolysed milk solids were prepared by the same process as specified in example 1.

The production process of these spreads was as follows.
First a premix was prepared by dispersing Purity LFS (starch) in water (15% solution w/w), while heating to 85°C and then holding for 40 minutes. After cooling down the solution to 65-70°C , BMP, Salt, Sorbate and hydrolysed milk solids are dissolved in the rest of the (boiling) water while thoroughly mixing. This mixture is cooled down to about 60°C, and added to the starch solution. Citric acid solution (20%) is added to adjust pH to 5.1. The fat-phase with the fat soluble ingredients is added to the aqueous phase, while mixing, and water soluble flavours are added.

The products comprised 16 mg LTP per 100 g product.

This premix is processed on a lab-scale micro votator with sequence A-A-A-C* with a throughput of 11kg/hr. The following cooling profile is used indicating the outlet temperatures of the A-units: A1=20°C, A2=10°C and A3=6°C with rotations set on 1000rpm. The inversion takes place in a 75ml C-unit with 2500rpm. The products are fat-continuous spreads.

The people in the taste panel compared the taste of the 3 fat-continuous spreads FCS-1, FCS-2 and FCS-3, by tasting the spread pure. According to the panel members, the FCS-3 spread (with plant sterolesters and hydrolysed milk solids) showed less cheesy and cardboard off-taste than the FCS-2 spread (hydrolysed milk solids).

## Example 3

A yoghurt was prepared with the product composition in wt% on total product as presented in table below. The hydrolysed milk solids were prepared by the same process as specified in example 1.

Yoghurt drink product composition

| Ingredient | Yoghurt drink 1 (Wt%) Comparison example | Yoghurt drink 2 (Wt%) Comparison example | Yoghurt drink 3 (Wt%) According to the invention |
|---|---|---|---|
| Skimmed milk | 60 | 60 | 60 |
| cream (40% fat) | 2 | 2 | 2 |
| sugar | 8 | 8 | 8 |
| tocopherol | 200 ppm | 200 ppm | 200 ppm |
| Hydrolysed milk solids | 0 | 1.5 | 1.5 |
| Sterolester of sunflower oil | 3 | 0 | 3 |
| water | Up to 99.8wt% | Up to 99.8wt% | Up to 99.8 wt% |
| total base | 99.80 | 99.80 | 99.80 |
| pre-culture | 0.20 | 0.20 | 0.20 |
| total yoghurt drink | 100.00 | 100.00 | 100.00 |

A premix was prepared comprising milk, cream and water at a temperature of about 60 °C. Tocopherols and sterolester was weighed and mixed with a spoon. The aqueous mixture was subjected to vigorous stirring in a Turrax™. While the Turrax™ was run at low speed of about 3500 rpm, the powder mix of sugar and hydrolysed milk solids was mixed in, followed by the sterolester composition. Mixing was continued at higher speed for some more minutes. The mixture was then heated to 75 °C and pasteurised. Next the mix was homogenised at 200 bar and cooled to fermentation temperature of about 43 °C.

A separately prepared yoghurt culture was used to inoculate the product. Fermentation was continued until a pH of about 4.3 was reached. The fermented product was stirred and homogenised at about 50 bar before it was filled into sterile containers. The product was cooled and stored at 5 °C.

Yoghurt drink 2 and 3 comprised 9.5 mg LTP per 100 g product.

The products were tasted by an expert panel. Yoghurt drink 3 was evaluated as best by the test panel. Especially with regard to off taste in comparison with Yoghurt drink 2, and expecially with regard to fatty mouthfeel in comparison with Yoghurt drink 1.

These results show that a combination of both hydrolysed milk solids and sterol esters is advantageous for both product taste and mouthfeel.

**Claims**

1. Food product comprising hydrolysed animal milk solids in which the hydrolysed milk solids contain tripeptides selected from the group comprising VPP, IPP, LPP, or a combination thereof and the product further comprises a sterol fatty acid ester, the amount of tripeptides VPP, IPP together being defined as LTP and being at least 0.5 mg/100 g, wherein [LTP] = [VPP] * 1.7[IPP]; [LTP], [VPP] and [IPP] being expressed in (mg/100 g).

2. Food product according to claim 1 which is an oil and water emulsion.

3. Food product according to any of the preceding claims, wherein the food product further comprises from 1 to 20 wt% sterol fatty acid ester.

4. Food product according to any of the preceding claims wherein the hydrolysed milk solids or tripeptides are obtained by enzymatic treatment of a substrate comprising casein or casein fragments.

5. Food product according to any of the preceding claims comprising at least 3.5 mg LTP per 100 g product.

6. Food product according to any of the preceding claims, further comprising live bacteria.

7. Composition as defined in any one of claims 1-6 for use in reducing of cholesterol levels in blood and/or blood pressure lowering.

8. Use of a sterol fatty acid ester to reduce off taste, especially cardboard off taste in a food product comprising hydrolysed milk solids wherein the milk is animal milk and milk solids comprising a peptide selected from the group comprising tripeptides VPP, IPP, LPP, or a combination thereof, wherein the amount of tripeptides VPP, IPP together is defined as LTP and is at least 0.5 mg/100 g.

9. Use according to claim 8 to reduce cheesy off taste.

10. Food product according to claim 1, wherein the food product is a drink comprising live cultures, hydrolysed casein, up to 5 wt% fat, from 1.5 to 4 wt% sterol fatty acid ester, milk protein, water, a sugar and preferably emulsifier.


**Patentansprüche**

1. Lebensmittelprodukt, umfassend hydrolysierte tierische Milchfeststoffe, in denen die hydrolysierten Milchfeststoffe Tripeptide enthalten, die aus der VPP, IPP, LPP oder eine Kombination davon umfassenden Gruppe ausgewählt sind, und das Produkt des Weiteren einen Sterolfettsäureester enthält, wobei die Menge der Tripeptide VPP, IPP zusammen als LTP definiert ist und mindestens 0,5 mg/100 g beträgt, worin [LTP] = [VPP] * 1,7 [IPP]; [LTP], [VPP] und [IPP] in (mg/100 g) ausgedrückt sind.

2. Lebensmittelprodukt nach Anspruch 1, das eine Emulsion von Öl und Wasser darstellt.

3. Lebensmittelprodukt nach einem der vorhergehenden Ansprüche, worin das Lebensmittelprodukt des Weiteren 1 bis 20 Gew.-% Sterolfettsäureester enthält.

4. Lebensmittelprodukt nach irgendeinem der vorhergehenden Ansprüche, worin die hydrolysierten Milchfeststoffe oder Tripeptide durch enzymatische Behandlung eines Substrats erhalten werden, das Casein oder Casein-Fragmente umfasst.

5. Lebensmittelprodukt nach irgendeinem der vorhergehenden Ansprüche, umfassend mindestens 3,5 mg LTP pro 100 g Produkt.

6. Lebensmittelprodukt nach irgendeinem der vorhergehenden Ansprüche, des Weiteren enthaltend lebende Bakterien.

7. Zusammensetzung der Definition nach irgendeinem der Ansprüche 1 bis 6 zur Verwendung bei der Verminderung des Cholesterinspiegels im Blut und/oder zur Herabsetzung des Blutdrucks.

8. Verwendung eines Sterolfettsäureesters zur Verminderung des Beigeschmacks, insbesondere des Beigeschmacks von Karton, in einem Lebensmittelprodukt, das hydrolysierte Milchfeststoffe enthält, worin die Milch tierische Milch darstellt und die Milchfeststoffe ein Peptid umfassen, ausgewählt aus der die Tripeptide VPP, IPP, LPP oder eine Kombination davon umfassenden Gruppe, wobei die Menge der Tripeptide VPP, IPP zusammen als LTP definiert ist und mindestens 0,5 mg/100 g beträgt.

9. Verwendung nach Anspruch 8 zur Verminderung des Käsebeigeschmacks.

10. Lebensmittelprodukt nach Anspruch 1, worin das Lebensmittelprodukt ein Getränk ist, das lebende Kulturen, hydrolysiertes Casein, bis zu 5 Gew.-% Fett, 1,5 bis 4 Gew.-% Sterolfettsäureester, Milchprotein, Wasser, einen Zucker und vorzugsweise ein Emulgiermittel umfasst.


**Revendications**

1. Produit alimentaire comprenant de la matière sèche obtenue après hydrolyse du lait animal dans lequel la matière sèche obtenue après hydrolyse du lait contient des tripeptides choisis dans le groupe comprenant VPP, IPP, LPP ou une combinaison de ceux-ci et le produit comprend en outre un ester d'acide gras de stérol, la quantité de tripeptides VPP, IPP conjoints étant définie par LTP et étant d'au moins 0,5 mg/100 g, où [LTP] = [VPP] * 1,7[IPP] ; [LTP], [VPP] et [IPP] étant exprimés en (mg/100 g).

2. Produit alimentaire selon la revendication 1, qui est une émulsion d'huile et d'eau.

3. Produit alimentaire selon l'une quelconque des revendications précédentes, dans lequel le produit alimentaire comprend en outre de 1 à 20 % en poids d'ester d'acide gras de stérol.

4. Produit alimentaire selon l'une quelconque des revendications précédentes, dans lequel la matière sèche obtenue après hydrolyse du lait ou les tripeptides sont obtenus par traitement enzymatique d'un substrat comprenant de la caséine ou des fragments de caséine.

5. Produit alimentaire selon l'une quelconque des revendications précédentes, comprenant au moins 3,5 mg de LTP pour 100 g de produit.

6. Produit alimentaire selon l'une quelconque des revendications précédentes, comprenant en outre des bactéries vivantes.

7. Composition telle que définie dans l'une quelconque des revendications 1 à 6 pour une utilisation dans la réduction du taux de cholestérol dans le sang et/ou l'abaissement de la tension artérielle.

8. Utilisation d'un ester d'acide gras de stérol pour réduire le mauvais goût, notamment le mauvais goût de carton dans un produit alimentaire comprenant de la matière sèche obtenue après hydrolyse du lait, dans laquelle le lait est un lait animal et la matière sèche du lait comprenant un peptide choisi dans le groupe comprenant les tripeptides VPP, IPP, LPP ou une combinaison de ceux-ci, dans laquelle la quantité de tripeptides VPP, IPP conjoints est définie par LTP et est d'au moins 0,5 mg/100 g.

9. Utilisation selon la revendication 8, pour réduire le mauvais goût de fromage.

10. Produit alimentaire selon la revendication 1, dans lequel le produit alimentaire est une boisson comprenant des cultures vivantes, de la caséine hydrolysée, jusqu'à 5 % en poids de matières grasses, de 1,5 à 4 % en poids d'ester d'acide gras de stérol, une protéine du lait, de l'eau, du sucre et de préférence un émulsifiant.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 821968 A
- EP 1365656 A
- WO 03055324 A
- WO 0355324 A
- WO 04098309 A
- WO 01034828 A

- EP 1231279 A
- WO 0134828 A
- EP 583074 A
- WO 04060073A1 A
- EP 594612 A
- WO 2004014141 A

**Non-patent literature cited in the description**

- **GERSTEIN et al.** *The Lancet,* 2000, vol. 355, 253-259
- **MIZUNO S et al.** *J Dairy Sci,* 2004, vol. 87, 3183-3188

- **NAKAMURA Y et al.** *J Dairy Sci,* 1995, vol. 78, 777-783
- **S.P. KOCHHAR.** Influence of Processing on Sterols of Edible Vegetable Oils. *Prog. Lipid Res.,* vol. 22, 161-188